# EUROPEAN PATENT APPLICATION

(11) **EP 2 839 831 A1**
(43) Date of publication of application: **25.02.2015**
(21) Application number: 13181057.4
(22) Date of filing: 20.08.2013
(51) Int. Cl.: A61K 31/22, A61K 45/00, A61K 31/365, A61K 31/366, A61K 31/40, A61K 31/404, A61K 31/4155, A61K 31/4402, A61K 31/454, A61K 31/505, A61K 31/5377, A61K 31/5513, A61P 25/28

(54) **Modulators of the unconventional secretory pathway for use in the treatment of Alexander disease**

(71) Applicant: Rheinische Friedrich-Wilhelms-Universität Bonn, 53113 Bonn (DE)
(72) Inventor: Glebov, Konstantin, 53127 Bonn (DE); Walter, Jochen, 53757 Sankt Augustin (DE)
(74) Representative: Michalski, Stefan

(57) **Abstract**

The present invention relates to modulators of the unconventional secretory pathway for use in the treatment of Alexander disease.

## Description

The present invention relates to modulators of the unconventional secretory pathway for use in the treatment of Alexander disease.

Alexander disease is a rare, slowly progressing and incurable neurodegenerative disorder that is attributed to mutations in glial fibrillary acidic protein (GFAP), with one of the main symptoms being a demyelination. While it is known that GFAP mutations are detrimental for the development of Alexander disease, the exact pathomechanism is still unknown. Glial fibrillary acidic protein is a type III intermediate filament that contains so-called head, rod and tail domains. Many of the disease-causing mutations are located in the so-called 2A rod domain. An analysis of prevalence of onset for Alexander disease revealed that mutations in the 2A rod region are responsible for most of the severe and early cases with infantile and juvenile onset of Alexander disease.

It has been suggested that the unconventional secretory pathway (USP) might play some role in neurodegenerative disease pathology such as Alzheimer's disease, as proteins associated specifically with neurodegenerative diseases can be secreted by unconventional means. For example the insulin-degrading enzyme (IDE), which is a ubiquitously expressed zinc-metalloprotease that degrades several pathophysiologically significant substrates such as the amyloid β-protein, is a protein that can be secreted into the extracellular space via the unconventional secretory pathway.

Currently, no cure or accepted course of treatment for Alexander disease is available. Therefore, the objective underlying the present invention was to provide means being usable in the treatment of Alexander disease.

The problem is solved by a modulator of the unconventional secretory pathway selected from the group comprising 3-hydroxy-3-methylglutaryl-Coenzyme A (HMG-CoA) reductase inhibitors, farnesyl transferase inhibitors, Rho-associated protein kinase inhibitors, geranylgeranyl transferase inhibitors, cells expressing the wild type form of glial fibrillary acidic protein (GFAP) gene and/or viruses comprising the Rab11, Rab27a, Rab27b, or Rab35 gene for use in the treatment of Alexander disease.

Surprisingly it was found that modulators of the unconventional secretory pathway are able to reverse the effect of Alexander disease-related mutations in glial fibrillary acidic protein (GFAP) on the secretion of insulin-degrading enzyme (IDE). Beneficially, the modulators according to the invention can be useful in the treatment of Alexander disease. The findings suggest that modulators of the unconventional secretory pathway provide a viable approach to control the unconventional secretion pathway in case of certain Alexander disease-causing GFAP mutations. Hence, the modulators of the unconventional secretory pathway can be used to alleviate the symptoms and prolong the life expectancy.

It is thought that the advantageous effects are derived due to an interaction between glial fibrillary acidic protein and the insulin-degrading enzyme. This interaction was shown to be affected by Alexander disease-related mutations in the 2A rod region of GFAP. These clinically relevant GFAP mutations by affecting the association between insulin-degrading enzyme (IDE) and GFAP thereby impair the unconventional secretory pathway in Alexander disease patients. Using the insulin-degrading enzyme as a marker for the unconventional secretory pathway, it could be shown that Alexander disease-related mutations in the 2A rod region of GFAP lead to a significant reduction in the secretion of insulin-degrading enzyme. This influence of Alexander disease-related mutations of GFAP can be reversed by the modulators of the unconventional secretory pathway, as the treatment of cells expressing the Alexander disease-related mutated variant of GFAP significantly improved the secretion of insulin-degrading enzyme (IDE).

Preferred modulators of the unconventional secretory pathway for use in the treatment of Alexander disease are HMG-CoA reductase inhibitors. As used herein, the term "HMG-CoA reductase inhibitor" refers to a compound that targets and inhibits the activity of the 3-hydroxy-3-methylglutaryl-Coenzyme A (HMG-CoA) reductase. HMG-CoA reductase is an enzyme of the mevalonate pathway, which provides cholesterol and other isoprenoids. In a preferred embodiment, the HMG-CoA reductase inhibitor is a statin. Preferred statins are selected from the group comprising lovastatin (INN), atorvastatin (INN), fluvastatin (INN), pitavastatin (INN), pravastatin (INN), rosuvastatin (INN) and/or simvastatin (INN). A most preferred statin is lovastatin. Lovastatin is the International Nonproprietary Name (INN) of a statin denoted [(1S,3R,7S,8S,8aR)-8-[2-[(2R,4R)-4-hydroxy-6-oxooxan-2-yl]ethyl]-3,7-dimethyl-1,2,3,7,8,8a-hexahydronaphthalen-1-yl] (2S)-2-methylbutanoate according to the IUPAC nomenclature. It could be shown that lovastatin can rescue the effect of the Alexander disease-causing mutation on the secretion of endogenous insulin-degrading enzyme (IDE).

The statins atorvastatin (INN), fluvastatin (INN), pitavastatin (INN), pravastatin (INN), rosuvastatin (INN) and simvastatin (INN) are denoted:
- (3*R*,5*R*)-7-[2-(4-fluorophenyl)-3-phenyl-4-(phenylcarbamoyl)-5-propan-2-ylpyrrol-1-yl]-3,5-dihydroxyheptanoic acid,
- (3*R*,5*S*,6*E*)-7-[3-(4-fluorophenyl)-1-(propan-2-yl)-1*H*-indol-2-yl]-3,5-dihydroxyhept-6-enoic acid,
- (3*R*,5*S*,6*E*)-7-[2-cyclopropyl-4-(4-fluorophenyl)quinolin-3-yl]-3,5-dihydroxyhept-6-enoic acid,
- (3*R*,5*R*)-3,5-dihydroxy-7-((1*R*,2*S*,6*S*,8*R*,8a*R*)-6-hydroxy-2-methyl-8-{[(2*S*)*-*2-methylbutanoyl]oxy}-1,2,6,7,8,8a-hexahydronaphthalen-1-yl)-heptanoic acid,
- (3*R*,5*S*,6*E*-7-[4-(4-fluorophenyl)-2-(*N-*methylmethanesulfonamido)-6-(propan-2-yl)pyrimidin-5-yl]-3,5-dihydroxyhept-6-enoic acid, and
- (1*S*,3*R*,7*S*,8*S*,8a*R*)-8-{2-[(2*R*,4*R*)-4-hydroxy-6-oxotetrahydro-2*H-*pyran-2-yl]ethyl}-3,7-dimethyl-1,2,3,7,8,8a-hexahydronaphthalen-1-yl 2,2-dimethylbutanoate,
   respectively, according to the IUPAC nomenclature.

Further modulators of the unconventional secretory pathway for use in the treatment of Alexander disease are farnesyl transferase inhibitors. As used herein, the term "farnesyl transferase inhibitor" refers to a class of drugs that target and inhibit the activity of a protein denoted farnesyl-transferase. This enzyme adds a fatty acid molecule to proteins such as the Ras protein. Farnesyl transferase inhibitors can be selected from the group comprising compounds tipifarnib (INN), lonafarnib (INN), and experimental substances BMS-214662, L778123, FTI-277, and L744832.

Tipifarnib (INN) and lonafarnib (INN) are denoted 6-[amino(4-chlorophenyl)(1-methyl-1*H-*imidazol-5-yl)methyl]-4-(3-chlorophenyl)-1-methylquinolin-2(1*H*)-one and 4-(2-(4-(8-Chloro-3,10-dibromo-6,11-dihydro-5H-benzo(5,6)cyclohepta(1,2-b)pyridin-11-yl)-1-piperidinyl)-2-oxoethyl)-1-piperidinecarboxamide, respectively, according to the IUPAC nomenclature.

The compounds BMS-214662, L778123, FTI-277, and L744832 are denoted:
- (R)-1-((1H-imidazol-5-yl)methyl)-3-benzyl-4-(thiophen-2-ylsulfonyl)-2,3,4,5-tetrahydro-1H-benzo[e][1,4]diazepine-7-carbonitrile,
- Benzonitrile,4-[[5-[[4-(3-chlorophenyl)-3-oxo-1-piperazinyl]methyl]-1H-imidazol-1-yl]methyl]-,monohydrochloride (9CI);4-((5-((4-(3-Chlorophenyl)-3-oxo-1-piperazinyl)methyl)-1H-imidazol-1-yl)methyl)benzonitrile hydrochloride,
- L-Methionine,N-[[5-[[(2R)-2-amino-3-mercaptopropyl]amino][1,1'-biphenyl]-2-yl]carbonyl]-,methyl ester, and
- propan-2-yl(2S)-2-[[(2S)-2-[(2S,3R)-2-[[(2R)-2-amino-3-sulfanylpropyl]amino]-3-methylpentoxy] -3-phenylpropanoyl] amino]-4-methylsulfonylbutanoate,
   respectively, according to the IUPAC nomenclature.

Further preferred modulators of the unconventional secretory pathway for use in the treatment of Alexander disease are Rho-associated protein kinase (ROCK) inhibitors. As used herein, the term "Rho-associated protein kinase inhibitor" refers to a class of drugs that target and inhibit the activity of a protein denoted Rho-associated protein kinase (ROCK). Rho-associated protein kinase (ROCK) is a serine/threonine kinase. Two ROCK isoforms ROCK1 and ROCK2 have been discovered as being effectors of the small GTPase RhoA. ROCKs have an amino-terminal kinase domain, a mid coiled-coil-forming region containing a Rho-binding domain, and carboxy-terminal cysteine-rich domain.

In an embodiment, the Rho-associated protein kinase (ROCK) inhibitors are selected from the group comprising:
- (1R,4r)-4-((R)-1-aminoethyl)-N-(pyridin-4-yl)cyclohexanecarboxamide dihydrochloride (Y-27632),
- *N*-[3-[[2-(4-Amino-1,2,5-oxadiazol-3--yl)-1-ethyl-1*H*-imidazo[4,5-*c*]pyridin-6-yl]oxy]phe-nyl]-4-[2-(4-morpholinyl)ethoxy]benzamide (GSK 269962),
- 4-[4-(Trifluoromethyl)phenyl]-*N*-(6--Fluoro-1*H*-indazol-5-yl)-2-methyl-6-oxo-1,4,5,6-tet-rahydro-3-pyridinecarboxamide (GSK 429286),
- (*S*)-(+)-2-Methyl-1-[(4-methyl-5-iso-quinolinyl)sulfonyl]-hexahydro-1*H*-1,4-diazepine (H 1152),
- (*S*)-(+)-4-Glycyl-2-methyl-1-[(4-met-hyl-5-isoquinolinyl)sulfonyl]-hexahydro-1*H*-1,4-diazepine (Glycyl-H 1152),
- 1-[(1,2-Dihydro-1-oxo-5-isoquinolin-yl)sulfonyl]hexahydro-1*H-*1,4-diazepine hydrochloride (HA 1100),
- (3*S*)-1-[[2-(4-Amino-1,2,5-oxadiazol--3-yl)-1-ethyl-1*H*-imidazo[4,5-*c*]pyridin-7-yl]carbo-nyl]-3-pyrrolidinamine (SB 772077B),
- *N*-[2-[2-(Dimethylamino)ethoxy]-4-(1-*H*-pyrazol-4-yl)phenyl-2,3-dihydro-1,4-benzodioxin--2-carboxamide (SR 3677), and/or
- fasudil.

Y-27632 is a ROCK inhibitor available as a dihydrochloride denoted (1R,4r)-4-((R)-1-aminoethyl)-N-(pyridin-4-yl)cyclohexanecarboxamide dihydrochloride or (+)-(R)-trans-4-(1-aminoethyl)-N-(4-pyridyl)cyclohexanecarboxamide dihydrochloride according to the IUPAC nomenclature. Fasudil is the International Nonproprietary Name (INN) of a selective RhoA/Rho kinase (ROCK) inhibitor denoted 5-(1,4-diazepane-1-sulfonyl)isoquinoline according to the IUPAC nomenclature.

The further ROCK inhibitors usually are denoted GSK 269962, GSK 429286, H 1152, Glycyl-H 1152, HA 1100, SB 772077B, and SR 3677.

Further modulators of the unconventional secretory pathway for use in the treatment of Alexander disease are geranylgeranyl transferase inhibitors. As used herein, the term "geranylgeranyl transferase inhibitor" refers to a class of drugs that target and inhibit the activity of a protein denoted geranylgeranyl transferase. Geranylgeranyl transferase postranslationally modifies proteins by adding a prenyl group, an isoprenoid lipid, to the carboxyl terminus of the target protein. Geranylgeranyltransferase inhibitors can be selected from the group comprising GGTI 297 and/or GGTI 298. GGTI 297 is the experimental name of a compound denoted *N*-[4-[2(*R*)-Amino-3-mercaptopropyl]amino-2-(1-naphthalenyl)benzoyl]-L-leucine trifluoroacetate salt. GGTI 297 inhibits RhoA prenylation leading to an inactivation of RhoA/ROCK. GGTI 298 is the experimental name of a compound denoted *N-*[4-[2(*R*)-Amino-3-mercaptopropyl]amino-2-(1-naphthalenyl)benzoyl]-L-leucine methyl ester trifluoroacetate salt. GGTI 298 inhibits the processing of geranylgeranylated Rap1A.

Further preferred modulators of the unconventional secretory pathway for use in the treatment of Alexander disease are cells expressing the wild type form of GFAP gene and/or viruses comprising the Rab11, Rab27a, Rab27b, or Rab35 gene.

Also cell-based approaches can provide a viable approach in the treatment of Alexander disease. Cells having undisturbed unconventional secretory pathway (USP) can replace or rescue patient faulty USP. Particularly, a Rab-containing virus or cells overexpressing the wild type form of GFAP gene are usable in therapy. Viral gene therapy most frequently uses non-replicating viruses to deliver therapeutic genes to cells with genetic malfunctions. Particularly the Ras-related protein Rab-35 and Rab11, Rab27a/b are usable, as for example those proteins are involved in the regulation of exosome secretion. Preferred is Rab-35.

A further aspect of the present invention relates to a pharmaceutical composition comprising as an active ingredient a modulator of the unconventional secretory pathway selected from the group comprising 3-hydroxy-3-methylglutaryl-Coenzyme A (HMG-CoA) reductase inhibitors, farnesyl transferase inhibitors, Rho-associated protein kinase inhibitors, geranylgeranyl transferase inhibitors, cells expressing the wild type form of glial fibrillary acidic protein (GFAP) gene and/or viruses comprising the Rab11, Rab27a, Rab27b, or Rab35 gene for use in the treatment of Alexander disease.

Preferred modulators of the unconventional secretory pathway are HMG-CoA reductase inhibitors. In a preferred embodiment, the pharmaceutical composition comprises as a HMG-CoA reductase inhibitor a statin. Preferred statins are selected from the group comprising lovastatin (INN), atorvastatin (INN), fluvastatin (INN), pitavastatin (INN), pravastatin (INN), rosuvastatin (INN) and/or simvastatin (INN). A most preferred statin is lovastatin.

Further preferred modulators of the unconventional secretory pathway are Rho-associated protein kinase (ROCK) inhibitors. In an embodiment of the pharmaceutical composition, the Rho-associated protein kinase (ROCK) inhibitors are selected from the group comprising Y-27632, GSK 269962, GSK 429286, H 1152, Glycyl-H 1152, HA 1100, SB 772077B, SR 3677 and/or fasudil.

Further modulators of the unconventional secretory pathway for use in the pharmaceutical composition are farnesyl transferase inhibitors and geranylgeranyl transferase inhibitors. Farnesyl transferase inhibitors can be selected from the group comprising compounds tipifarnib (INN), lonafarnib (INN), and experimental substances BMS-214662, L-778,123, FTI-277 and/or L-744,832. Geranylgeranyl transferase inhibitors can be selected from the group comprising GGTI 297 and/or GGTI 298.

Further preferred modulators of the unconventional secretory pathway for use in the pharmaceutical composition are cells expressing the wild type form of GFAP gene and/or viruses comprising the Rab11, Rab27a, Rab27b, or Rab35 gene.

The pharmaceutical composition can comprise a modulator of the unconventional secretory pathway according to the invention as an active ingredient, a pharmaceutically acceptable carrier and optionally other therapeutic ingredients or adjuvants. The present invention hence also relates to a pharmaceutical composition wherein the composition comprises as an active ingredient a modulator of the unconventional secretory pathway according to the invention, and a pharmaceutically acceptable carrier for use in the treatment of Alexander disease.

The pharmaceutical carrier can be, for example, a solid, liquid, or gas. Suitable carriers and adjuvants can be solid or liquid and correspond to the substances ordinarily employed in formulation technology for pharmaceutical formulations. For example, water, glycols, oils, alcohols and the like may be used to form liquid preparations such as solutions. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen. The compositions can be suitable for oral, dermal, rectal, topical, and parenteral administration. Parenteral administration includes subcutaneous, intramuscular, and intravenous administration, and particularly includes intracranial injections and cerebral shunts.

Compositions suitable for parenteral administration may be prepared as solutions or suspensions in water. Compositions suitable for injectable use include sterile aqueous solutions or dispersions. The pharmaceutical compositions may be conveniently presented in unit dosage form and prepared by any of the methods well known in the art of pharmacy. The pharmaceutical composition may be produced under sterile conditions using standard pharmaceutical techniques well known to those skilled in the art.

The present invention also relates to the use of a modulator of the unconventional secretory pathway selected from the group comprising 3-hydroxy-3-methylglutaryl-Coenzyme A (HMG-CoA) reductase inhibitors, farnesyl transferase inhibitors, Rho-associated protein kinase inhibitors, geranylgeranyl transferase inhibitors, cells expressing the wild type form of glial fibrillary acidic protein (GFAP) gene and/or viruses comprising the Rab11, Rab27a, Rab27b, or Rab35 gene for the manufacture of a medicament for the treatment of Alexander disease.

Preferably usable modulators of the unconventional secretory pathway are HMG-CoA reductase inhibitors. In a preferred embodiment, the usable HMG-CoA reductase inhibitor is a statin. Preferred statins are selected from the group comprising lovastatin (INN), atorvastatin (INN), fluvastatin (INN), pitavastatin (INN), pravastatin (INN), rosuvastatin (INN) and/or simvastatin (INN). A most preferred statin is lovastatin.

Further preferably usable modulators of the unconventional secretory pathway are Rho-associated protein kinase (ROCK) inhibitors. In an embodiment, the usable Rho-associated protein kinase (ROCK) inhibitors are selected from the group comprising Y-27632, GSK 269962, GSK 429286, H 1152, Glycyl-H 1152, HA 1100, SB 772077B, SR 3677 and/or fasudil.

Further usable modulators of the unconventional secretory pathway for use in the pharmaceutical composition are farnesyl transferase inhibitors and geranylgeranyl transferase inhibitors. Farnesyl transferase inhibitors can be selected from the group comprising compounds tipifarnib (INN), lonafarnib (INN), and experimental substances BMS-214662, L-778,123, FTI-277 and/or L-744,832. Geranylgeranyl transferase inhibitors can be selected from the group comprising GGTI 297 and/or GGTI 298.

Further preferably usable modulators of the unconventional secretory pathway for use in the pharmaceutical composition are cells expressing the wild type form of GFAP gene and/or viruses comprising the Rab11, Rab27a, Rab27b, or Rab35 gene.

A further aspect of the present invention relates to a method of treating Alexander disease, the method comprising administering to a subject a therapeutically effective amount of a modulator of the unconventional secretory pathway selected from the group comprising 3-hydroxy-3-methylglutaryl-Coenzyme A (HMG-CoA) reductase inhibitors, farnesyl transferase inhibitors, Rho-associated protein kinase inhibitors, geranylgeranyl transferase inhibitors, cells expressing the wild type form of glial fibrillary acidic protein (GFAP) gene and/or viruses comprising the Rab11, Rab27a, Rab27b, or Rab35 gene.

The term "therapeutically effective amount" is used herein to mean an amount or dose sufficient to cause an improvement in a clinically significant condition in the subject.

In embodiments the method of treating Alexander disease refers to administering HMG-CoA reductase inhibitors, particularly a statin. Preferred statins are selected from the group comprising lovastatin (INN), atorvastatin (INN), fluvastatin (INN), pitavastatin (INN), pravastatin (INN), rosuvastatin (INN) and/or simvastatin (INN). A most preferred statin is lovastatin.

In another embodiment, the method of treating Alexander disease refers to administering Rho-associated protein kinase (ROCK) inhibitors selected from the group comprising Y-27632, GSK 269962, GSK 429286, H 1152, Glycyl-H 1152, HA 1100, SB 772077B, SR 3677 and/or fasudil. Further modulators of the unconventional secretory pathway for use in the method of treating Alexander disease are farnesyl transferase inhibitors and geranylgeranyl transferase inhibitors. Farnesyl transferase inhibitors can be selected from the group comprising compounds tipifarnib (INN), lonafarnib (INN), and experimental substances BMS-214662, L-778,123, FTI-277 and/or L-744,832. Geranylgeranyl transferase inhibitors can be selected from the group comprising GGTI 297 and/or GGTI 298. Further modulators of the unconventional secretory pathway for use in the method of treating Alexander disease are cells expressing the wild type form of GFAP gene and/or viruses comprising the Rab11, Rab27a, Rab27b, or Rab35 gene.

Unless otherwise defined, the technical and scientific terms used herein have the same meaning as commonly understood by one of ordinary skill in the art to which this invention belongs.

The Examples which follow serve to illustrate the invention in more detail but do not constitute a limitation thereof.

The figures show:

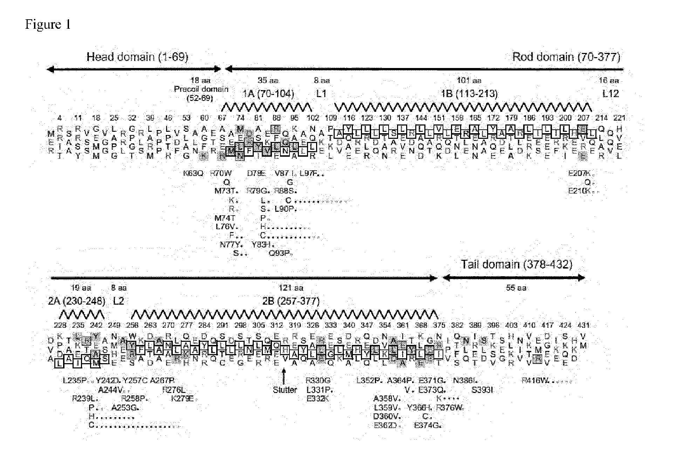

- Figure 1: The schematic representation of the GFAP organisation with the indicated mutations, which are responsible for the development of Alexander disease.

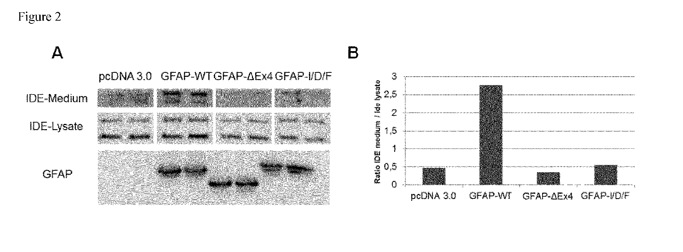

- Figure 2: The effect of Alexander disease causing mutations of GFAP on the secretion of IDE in COS-7 cells. Figure 2A shows a Western immunoblot analysis of IDE detected in the cell lysates and conditioned media after transfection of the cells with GFAP wild type (GFAP-WT), the Alexander disease causing mutations GFAP-ΔEx4 and GFAP-I/D/F. Figure 2B shows the respective quantification of IDE secretion given as the ratio of IDE in the medium to IDE in the lysates.

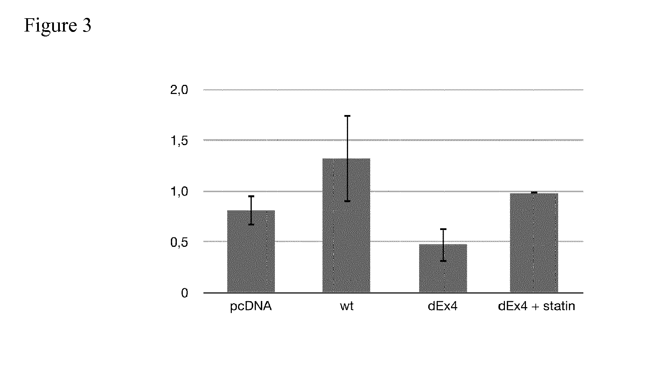

- Figure 3: The quantification of IDE secretion given as the ratio of IDE in the medium to IDE in the lysates after Western immunoblot analysis of IDE detected in the cell lysates and conditioned media after transfection of COS-7 cells with GFAP wild type (GFAP-WT), Alexander disease-causing GFAP mutation (GFAP-ΔEx4), and the effect of lovastatin (GFAP-ΔEx4 + statin).

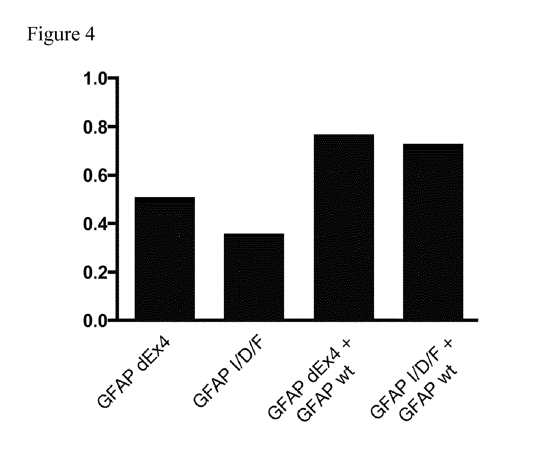

- Figure 4: The quantification of IDE secretion given as the ratio of IDE in the medium to IDE in the lysates of cells transfected with Alexander disease causing mutations GFAP-ΔEx4 or GFAP-I/D/F, and of probes of GFAP-ΔEx4 or GFAP-I/D/F transfected cells, to which were added cells transfected with GFAP wild type (GFAP-ΔEx4 + GFAP-wt) and (GFAP-I/D/F + GFAP-wt).

### Examples

### Materials and Methods:

### Cell Lines and Cell Culture

COS-7 cells, a fibroblast-like cell line from monkey kidney tissue (ATCC, USA), were cultured in Dulbecco's Modified Eagle Medium (DMEM) (Life Technologies) containing 10% Fetal calf serum (FCS) (Life Technologies) and 1% Penicillin/Streptomycin (PS) (Life Technologies) (DMEM +/+) and splitted into new dishes (Coming). For all experiments cells were counted by using a Neubauer counting chamber. To estimate cell number (cells/ml), the average number from four big squares of the Neubauer chamber was multiplied by 10'000 and the cell suspension was diluted to the desired cell concentration.

Primary cultures of astrocytes were obtained from E18 mouse embryos by brief trypsinzation of meninges-free brains with subsequent mechanical separation. Cell suspension was seeded in T-75 flasks and grown in DMEM containing 10% FCS with 1% PS for at least 14 days.

### Transient transfection of COS-7 cells

COS-7 were seeded on coverslips 24 hours before the transfection procedure. Transfection was done using Lipofectamine 2000 (Life Technologies) and according to the manufacturer guidelines. After 48 hours cells were fixed and subjected to immunofluorescent labeling procedure as described below.

### Immunocytochemistry

Cells were fixed with 4% paraformaldehyde (PFA), washed, permeabilized in 0.25% TritonX-100 (Roth) in phosphate buffered saline (PBS, 140 mM NaCl, 10 mM NaH₂PO₄, 1.75 mM KH₂PO₄, 2.5 mM KCl, pH 7.4), and blocked in 10% Bovine serum albumin (BSA, Roth) in PBS for 1 hour. The cells were then incubated with the appropriate primary antibodies IDE (rb) (1:300, Abcam), or GFAP (1:300, Cell Cignalling), for 1 hour in 5% BSA/PBS, washed, and then incubated in secondary antibodies for 1 hour (AlexaFluor 488, 546, 647 (Life Technologies), and Phalloidin:TRITC (1 mg/ml) (Sigma)) in 3% BSA/PBS.

### Protein extraction and Western immunoblotting

Cells were washed with PBS, resuspended in 800 µl lysis buffer (150 mM NaCl, 10 mM Tris pH 7.5, 1% Igepal, 5 mM EDTA), homogenized using a syringe with 23G needle and centrifuged at 13,200 rpm for 15 min at 4°C. The supernatant was boiled with loading buffer (5x SDS-Loading buffer: 250 mM TrisHCl pH 6.8, 30% Glycerin, 10% SDS, 5% β-mercaptoethanol, 0.02% Bromphenol blue).

Proteins were separated with SDS-PAGE (10-20% SDS polyacrylamide gels, Invitrogen). After SDS-PAGE separation proteins were transferred onto nitrocellulose membranes (Whatman Protran) by blotting at 180 V for 2 h and detected with the indicated primary antibodies and HRP conjugated secondary antibodies by enhanced chemiluminescence reagent (GE Healthcare). Signals were quantified with an ECL imager (Biorad) and QuantityOne software.

### Secretion assay

COS-7 cells transfected with pcDNA or GFAP variants were seeded on 6 well plates and 48 hours after transfection were washed with DMEM and 800 µl of fresh serum free DMEM was added. 12 to 16 hours later the conditioned medium was collected into 1.5 ml tubes and potential cell debris were removed by centrifugation at 1500 rcf for 10 minutes at 4°C. The remaining cells were harvested from dishes and corresponding lysates and samples of conditioned medium were prepared as described above.

### Imaging

Cell images were obtained on a Zeiss Axiovert 200M microscope equipped with a Apotome 2 and a CCD camera using a PlanApo 63x (1.40 N.A.) oil-immersion objective. For Apotome 2 aquired images four image avaregaing per z-plane was used. Images were captured and analyzed using AxioVision v4.8.2.0, Image J and Photoshop CS4 Extended software.

### Statistics

Statistical tests were performed using the student's t-test. Data are means ± SD, n= 2 independent treatments. P values less than 0.05 were considered as significant; whereas P > 0.05 means data are not significantly different.

### Example 1: Association of endogenous GFAP and IDE in primary astrocytes

The association of glial fibrillary acidic protein (GFAP) and endogenous insulin-degrading enzyme (IDE) was determined in primary astrocytes. Primary mouse astrocytes were obtained from E18 mouse embryos and after cultivation immunocytochemically stained for endogenous IDE and GFAP using IDE (1:300, Abcam) and GFAP (1:300, Cell Cignalling) specific antibodies.

Immunofluorescence microscopy of the primary mouse astrocytes showed a detection of endogenous IDE and GFAP, wherein IDE was mainly distributed as punctate structures in the cytosol whereas GFAP appeared as filamentous structures. A double staining for IDE and GFAP revealed an association of both proteins, as both proteins were detectable in the cytosol and IDE was associated with the GFAP filaments.

These data show that the glial fibrillary acidic protein (GFAP) associates with endogenous insulin-degrading enzyme (IDE) in primary mouse astrocytes.

### Example 2: Association of endogenous GFAP and IDE in COS-7 cells

To analyze whether association exists in further cells, COS-7 cells were used for transiently co-transfection of a generated IDE construct comprising the 1,019 amino acids of the mouse wild type and a C-terminal C-myc tag (IDE-WT-Myc) and a generated human wild type GFAP construct (GFAP-WT) comprising the complete head, rod and tail structure of 432 amino acids as shown in Figure 1. 48 hours after the transfection the cells were subjected to immunostaining with IDE (1:300, Abcam) and GFAP (1:300, Cell Cignalling) specific antibodies. For control, F-actin was visualized with Phalloidin:TRITC. Immunofluorescence microscopy of the primary mouse astrocytes showed that overexpressed GFAP formed an extensive intermediate filament network in the COS-7 cells that was similar to the GFAP expression pattern observed in the primary astrocytes in Example 1. Again, IDE was mainly distributed as punctate structures in the cytosol. A double staining for IDE and GFAP showed an association between IDE and GFAP in the full specter of filament assemblies found in COS-7 cells, from the thick bundles to the fine filaments.

These results suggest that IDE associates with GFAP in COS-7 cells in a similar manner as in primary mouse astrocytes.

### Example 3: Effect of mutations in the 2A rod domain in GFAP on the association with IDE

COS-7 cells were used for analysis of the protein-protein interaction of the GFAP constructs having the mutation causing Alexander disease and IDE. GFAP constructs having a mutation in the 2A rod domain causing Alexander disease (AxD) were generated and analysed for the association with IDE. The first GFAP construct had a C→G exchange 3 bp downstream of exon 4 (GFAP-ΔEx4). This mutation affects the transcription and the product was shorter because of the missing exon 4 (Flint et al., Hum Mutat 33:1141-1148, 2012). The second GFAP construct had an 8 bp deletion accompanied by a 3 bp insertion (1292 to 1299 bp). Such double mutation results in a frameshift at the C-terminus of GFAP(GFAP-I/D/F). Thereby the second mutation produced an elongated GFAP with additional 11 amino acids. The COS-7 cells were co transfected with a combination of IDE-WT-Myc and GFAP-ΔEx4 or of IDE-WT-Myc and GFAP-I/D/F as described above and after 48 hours of overexpression detected with Myc and GFAP specific antibodies.

Immunofluorescence microscopy of the COS-7 cells transiently co-transfected with IDE-WT-Myc and GFAP-ΔEx4 showed that the expression of the GFAP-ΔEx4 variant produced small dense spike-like structures. Also, no association between the GFAP-ΔEx4 variant and IDE was observed. This finding suggests that exon 4 of the glial fibrillary acidic protein (GFAP) contains a motif that is responsible for IDE and GFAP association.

Immunofluorescence microscopy of the COS-7 cells transiently co-transfected with IDE-WT-Myc and GFAP-I/D/F showed that the overexpression of the second mutated variant GFAP-I/D/F resulted in a formation of dense fibers of GFAP located mainly in the perinuclear region, with high IDE association. Since in GFAP-I/D/F variant potential association motif for IDE is unaffected, it can be suggested that wrong GFAP polymerization affected IDE/GFAP association.

In summary, these findings show that mutations in glial fibrillary acidic protein (GFAP) have an effect on the association with endogenous insulin-degrading enzyme (IDE).

### Example 4: Determination of the effect of Alexander disease-causing variants of GFAP on IDE release

To test whether the release of insulin-degrading enzyme (IDE) containing exosomes is affected by Alexander disease (AxD) causing variants of GFAP, COS-7 cells were transfected with GFAP-WT, GFAP-ΔEx4, and GFAP-I/D/F as described in Example 3. A secretion assay was carried out as described above. As a control pcDNA3.0 vector (Life Technologies) was used. The cells were transfected with the GFAP variants and incubated for 16 hours in DMEM+/+ and in additional step for 8 h in DMEM-/-. IDE was then detected in the cell lysates and conditioned media by Western immunoblotting. A quantification of the IDE secretion was done by ECL imaging.

The figure 2A shows the Western immunoblot analysis of IDE detected in the cell lysates and the conditioned media after the transfection. The figure 2B shows the quantification of the IDE secretion given as the ratio of IDE in the medium to IDE in the lysates. It can be taken from figure 2 that the overexpression of the GFAP-WT resulted in a stronger release of IDE, as the secretion increased in cells transfected with GFAP-WT in comparison to cells transfected with the control pcDNA 3.0. Both mutations strongly affected the efficiency of the IDE secretion. It can be taken from figure 2B that the mutations in GFAP disrupt the secretion of IDE.

From the results of the secretion assay experiment it can be concluded that GFAP modulates the release of IDE from COS-7 cells.

### Example 5: Effect of lovastatin on Alexander disease-affected secretion of IDE

The effect of statins on the secretion of IDE disturbed by Alexander disease-causing mutation in the 2A rod region of GFAP was determined by cell transfection and pharmacological treatment in COS-7 cells.

Cells were seeded in 6 well plates day before the transfection to a 50% confluence in DMEM medium supplemented with 10% FCS/1% PS. Next day GFAP variants were transfected using Lipofectamine (Life Technologys) according to the manufacturer's instructions. The COS-7 cells were transfected with GFAP wild type (GFAP-WT) and the Alexander disease causing mutation in the 2A rod region of GFAP (GFAP-ΔEx4) as described in Example 3. As a control pcDNA3.0 vector (Life Technologies) was used. 36 hours post transfection, the cells were treated either with DMSO (vehicle) or 5 µM lovastatin (Sigma Aldrich) for 15 hours. Conditioned media were collected and centrifuged (1000x g, 10 min, 4°C). Cells were lysed in ice-cold RIPA lysis buffer (pH 7.5). Proteins were separated by sodium dodecyl sulfate-polyacrylamide gel electrophoresis (SDS-PAGE), transferred to nitrocellulose membranes (Whatman), and detected by Western immunoblotting using enhanced chemiluminescence detection (ChemiDoc XRS, Bio-Rad). Signals were quantified with Quantity One Software (Bio-Rad).

The Figure 3 shows the quantification of IDE secretion given as the ratio of IDE in the medium to IDE in the lysates after Western immunoblot analysis of IDE detected in the cell lysates and conditioned media after transfection of the cells with GFAP wild type (GFAP-WT), the Alexander disease causing mutation in the 2A rod region of GFAP (GFAP-ΔEx4) and GFAP-ΔEx4 and lovastatin (GFAP-ΔEx4 + statin). It can be taken from figure 3 that while the Alexander disease causing GFAP mutation GFAP-ΔEx4 decreased the unconvential secretion of the IDE-positive exosomes in comparison to GFAP wt, the addition of lovastatin strongly improved the affected secretion, almost to a 100% increase compared to GFAP-ΔEx4.

These results show that statins can rescue the effect of the Alexander disease-causing mutation on the secretion of endogenous insulin-degrading enzyme (IDE).

### Example 6: Effect of cell-based therapy on Alexander disease-affected secretion of IDE

The effect of cells expressing the wild type variant of GFAP on the unconventional secretion disturbed by Alexander disease-related mutations of GFAP was determined by cell transfection and treatment in COS-7 cells.

COS-7 cells were transfected either with AxD-causing variants GFAP-ΔEx4 and GFAP-I/D/F or wild type GFAP as described in example 3. 24 hours later cells expressing wild type GFAP were added to cells expressing the Alexander disease-causing forms of GFAP in a 1:1 ratio. 48 hours after transfections conditioned media were collected and processed as described above. IDE was detected by Western immunoblotting using enhanced chemiluminescence detection (ChemiDoc XRS, Bio-Rad). Signals were quantified with ImageJ (NIH).

The Figure 4 shows the quantification of IDE secretion given as the ratio of IDE in the medium to IDE in the lysates after Western immunoblot analysis of cells transfected with Alexander disease causing mutations GFAP-ΔEx4 or GFAP-I/D/F, and of probes of cells transfected with Alexander disease causing mutations GFAP-ΔEx4 or GFAP-I/D/F, to which were added cells transfected with GFAP wild type (GFAP-ΔEx4 + GFAP-wt) and (GFAP-I/D/F + GFAP-wt). It can be taken from the Figure 4 that the addition of cells overexpressing GFAP wild type, led to a 50% increase (dEx4) and in case of I/D/F mutation almost to a 100% increase in release of exosomes.

This suggests that a cell-based therapy could provide a viable approach to control the unconventional secretion pathway in case of certain Alexander disease-causing GFAP mutations.

## Claims

1. A modulator of the unconventional secretory pathway selected from the group comprising 3-hydroxy-3-methylglutaryl-Coenzyme A (HMG-CoA) reductase inhibitors, farnesyl transferase inhibitors, Rho-associated protein kinase inhibitors, geranylgeranyl transferase inhibitors, cells expressing the wild type form of glial fibrillary acidic protein (GFAP) gene and/or viruses comprising the Rab11, Rab27a, Rab27b, or Rab35 gene for use in the treatment of Alexander disease.

2. The modulator according to claim 1, wherein the HMG-CoA reductase inhibitor is a statin, preferably selected from the group comprising lovastatin, atorvastatin, fluvastatin, pitavastatin, pravastatin, rosuvastatin and/or simvastatin.

3. The modulator according to claim 2, wherein the statin is lovastatin.

4. The modulator according to claim 1, wherein the Rho-associated protein kinase inhibitors are selected from the group comprising (1R,4r)-4-((R)-1-aminoethyl)-N-(pyridin-4-yl)cyclohexanecarboxamide dihydrochloride (Y-27632), *N*-[3-[[2-(4-Amino-1,2,5-oxadiazol-3--yl)-1-ethyl-1*H*-imidazo[4,5-*c*]pyridin-6-yl]oxy]phe-nyl]-4-[2-(4-morpholinyl)ethoxy]-benzamide (GSK 269962), 4-[4-(Trifluoromethyl)phenyl]-*N*-(6--Fluoro-1*H*-indazol-5-yl)-2-methyl-6-oxo-1,4,5,6-tet-rahydro-3-pyridinecarboxamide (GSK 429286), (*S*)-(+)-2-Methyl-1-[(4-methyl-5-iso-quinolinyl)sulfonyl]-hexahydro-1*H-*1,4-diazepine (H 1152), (*S*)-(+)-4-Glycyl-2-methyl-1-[(4-met-hyl-5-isoquinolinyl)sulfonyl]-hexahydro-1*H*-1,4-diazepine (Glycyl-H 1152), 1-[(1,2-Dihydro-1-oxo-5-isoquinolin-yl)sulfonyl]hexahydro-1*H-*1,4-diazepine hydrochloride (HA 1100), (3*S*)-1-[[2-(4-Amino-1,2,5-oxadiazol-3-yl)-1-ethyl-1*H-*imidazo[4,5-*c*]pyridin-7-yl]carbo-nyl]-3-pyrrolidinamine (SB 772077B), *N*-[2-[2-(Dimethylamino)ethoxy]-4-(1-*H-*pyrazol-4-yl)phenyl-2,3-dihydro-1,4-benzodioxin--2-carboxamide (SR 3677), and/or fasudil.

5. A pharmaceutical composition comprising as an active ingredient a modulator of the unconventional secretory pathway selected from the group comprising 3-hydroxy-3-methylglutaryl-Coenzyme A (HMG-CoA) reductase inhibitors, farnesyl transferase inhibitors, Rho-associated protein kinase inhibitors, geranylgeranyl transferase inhibitors, cells expressing the wild type form of glial fibrillary acidic protein (GFAP) gene and/or viruses comprising the Rab11, Rab27a, Rab27b, or Rab35 gene for use in the treatment of Alexander disease.

6. The pharmaceutical composition according to claim 5, wherein the HMG-CoA reductase inhibitor is a statin, preferably selected from the group comprising lovastatin, atorvastatin, fluvastatin, pitavastatin, pravastatin, rosuvastatin and/or simvastatin.

7. The pharmaceutical composition according to claim 6, wherein the statin is lovastatin.

8. The pharmaceutical composition according to claim 5, wherein the Rho-associated protein kinase inhibitors are selected from the group comprising Y-27632, GSK 269962, GSK 429286, H 1152, Glycyl-H 1152, HA 1100, SB 772077B, SR 3677 and/or fasudil.

9. Use of a modulator of the unconventional secretory pathway selected from the group comprising 3-hydroxy-3-methylglutaryl-Coenzyme A (HMG-CoA) reductase inhibitors, farnesyl transferase inhibitors, Rho-associated protein kinase inhibitors, geranylgeranyl transferase inhibitors, cells expressing the wild type form of glial fibrillary acidic protein (GFAP) gene and/or viruses comprising the Rab11, Rab27a, Rab27b, or Rab35 gene for the manufacture of a medicament for the treatment of Alexander disease.

10. The use according to claim 9, wherein the HMG-CoA reductase inhibitor is a statin, preferably selected from the group comprising lovastatin, atorvastatin, fluvastatin, pitavastatin, pravastatin, rosuvastatin and/or simvastatin, particularly lovastatin.

11. The use according to claim 9, wherein the Rho-associated protein kinase inhibitors are selected from the group comprising Y-27632, GSK 269962, GSK 429286, H 1152, Glycyl-H 1152, HA 1100, SB 772077B, SR 3677 and/or fasudil.

12. A method of treating Alexander disease, the method comprising administering to a subject a therapeutically effective amount of a modulator of the unconventional secretory pathway selected from the group comprising 3-hydroxy-3-methylglutaryl-Coenzyme A (HMG-CoA) reductase inhibitors, farnesyl transferase inhibitors, Rho-associated protein kinase inhibitors, geranylgeranyl transferase inhibitors, cells expressing the wild type form of glial fibrillary acidic protein (GFAP) gene and/or viruses comprising the Rab11, Rab27a, Rab27b, or Rab35 gene.

13. The method according to claim 12, wherein the HMG-CoA reductase inhibitor is a statin, preferably selected from the group comprising lovastatin, atorvastatin, fluvastatin, pitavastatin, pravastatin, rosuvastatin and/or simvastatin, particularly lovastatin.

14. The method according to claim 12, wherein the Rho-associated protein kinase inhibitors are selected from the group comprising Y-27632, GSK 269962, GSK 429286, H 1152, Glycyl-H 1152, HA 1100, SB 772077B, SR 3677 and/or fasudil.
